# EUROPEAN PATENT SPECIFICATION

(11) **EP 3 665 186 B1**
(45) Date of publication and mention of the grant of the patent: **04.10.2023**
(21) Application number: 18762922.5
(22) Date of filing: 03.08.2018
(51) Int. Cl.: C07K 14/005

(54) **RECOMBINANT PROTEIN VP60 AND PROCEDURE FOR ITS PRODUCTION AND PURIFICATION**
REKOMBINANTES PROTEIN VP60 UND VERFAHREN FÜR SEINE PRODUKTION UND REINIGUNG
PROTÉINE RECOMBINANTE VP60 ET PROCÉDURE POUR SA PRODUCTION ET PURIFICATION

(30) Priority: 07.08.2017 IT 201700091348
(43) Date of publication of application: 17.06.2020
(73) Proprietor: Istituto Zooprofilattico Sperimentale Dell'Umbria E Delle Marche, 06126 Perugia (PG) (IT)
(72) Inventor: DE GIUSEPPE, Antonio, 06126 Perugia (PG) (IT)
(74) Representative: Brunacci, Marco
(86) International application number: PCT/IB2018/055855
(87) International publication number: WO 2019/030630

(56) References cited:
- EP-A2- 0 704 529
- DONG-KUN YANG ET AL: "Rabbit Hemorrhagic Disease Virus Variant Recombinant VP60 Protein Induces Protective Immunogenicity", JOURNAL OF MICROBIOLOGY AND BIOTECHNOLOGY., vol. 25, no. 11, 28 November 2015 (2015-11-28), pages 1960-1965, XP055462009, KR ISSN: 1017-7825, DOI: 10.4014/jmb.1504.04002
- GUO HUIMIN ET AL: "Self-assembly of virus-like particles of rabbit hemorrhagic disease virus capsid protein expressed inEscherichia coliand their immunogenicity in rabbits", ANTIVIRAL RESEARCH, vol. 131, 23 April 2016 (2016-04-23), pages 85-91, XP029570505, ISSN: 0166-3542, DOI: 10.1016/J.ANTIVIRAL.2016.04.011
- XUEXING ZHENG ET AL: "Development of a VLP-based vaccine in silkworm pupae against rabbit hemorrhagic disease virus", INTERNATIONAL IMMUNOPHARMACOLOGY, vol. 40, 1 November 2016 (2016-11-01), pages 164-169, XP055461655, NL ISSN: 1567-5769, DOI: 10.1016/j.intimp.2016.08.016
- S LAURENT ET AL: "Structural, antigenic and immunogenic relationships between European brown hare syndrome virus and rabbit haemorrhagic disease virus.", JOURNAL OF GENERAL VIROLOGY., vol. 78, no. 11, 1 November 1997 (1997-11-01), pages 2803-2811, XP055461216, GB ISSN: 0022-1317, DOI: 10.1099/0022-1317-78-11-2803
- GIANTONELLA PUGGIONI ET AL: "The new French 2010 Rabbit Hemorrhagic Disease Virus causes an RHD-like disease in the Sardinian Cape hare (Lepus capensis mediterraneus)", VETERINARY RESEARCH, BIOMED CENTRAL LTD, LONDON, UK, vol. 44, no. 1, 7 October 2013 (2013-10-07), page 96, XP021162936, ISSN: 1297-9716, DOI: 10.1186/1297-9716-44-96
- G. LE GALL-RECUL? ET AL: "Phylogenetic analysis of rabbit haemorrhagic disease virus in France between 1993 and 2000, and the characterisation of RHDV antigenic variants", ARCHIVES OF VIROLOGY, vol. 148, no. 1, 1 January 2003 (2003-01-01), pages 65-81, XP055461919, AT ISSN: 0304-8608, DOI: 10.1007/s00705-002-0908-1
- NAGESHA H S ET AL: "SELF-ASSEMBLY, ANTIGENICITY, AND IMMUNOGENECITY OF THE RABBIT HAEMORRHAGIC DISEASE VIRUS (CZECHOSLOVAKIAN STRAIN V-351) CAPSID PROTEIN EXPRESSED IN BACULOVIRUS", ARCHIVES OF VIRO, SPRINGER WIEN, AT, vol. 140, no. 6, 1 June 1995 (1995-06-01), pages 1095-1108, XP009075295, ISSN: 0304-8608, DOI: 10.1007/BF01315418

## Description

### Technical Field

The present invention relates to a procedure for the production and purification of a recombinant VP60 protein from a Rabbit Hemorrhagic Disease Virus.

### Background Art

To date, the need is particularly felt to manage the spread of rabbit haemorrhagic disease virus (RHDV) from a health and environmental point of view.

This syndrome is caused by a Calicivirus, belonging to the family Caliciviridae, genus Lagovirus.

The Calcivirus viral genome comprises a single strand of RNA having a length between 7.5 kb and 8 kb.

As is known, Calciviruses have a capsid consisting of a single capsidic protein, the so-called VP60 (viral protein 60) having a molecular weight of about 60 kDa and a diameter of between 35 and 40 nm.

The capsidic protein VP60 of RHDV self-assembles spontaneously to form so-called "Virus-Like Particles" (VLPs) which, as is known, are particles without genetic material but morphologically and antigenically structured in the same way as mature virions.

The spread of RHDV syndrome occurs via the oral-nasal and conjunctival route, either through direct contact between infected animals or passively with convalescent animals.

Due to the high environmental resistance of the virus, the disease can also be indirectly transmitted, e.g. through contaminated feed, litter or fur, or alternatively through ingestion of contaminated food or water.

As mentioned above, RHDV is very stable and persists in the environment, in fact, it can survive in rabbit carcasses for at least 3 months, while the virus exposed directly to environmental weather conditions survives for less than 1 month.

Numerous studies report that morbidity is between 90-100%, while mortality is usually between 40% and 90%.

It is easy to appreciate how the strong spread of RHDV syndrome together with the high infectivity, mortality and resistance to environmental conditions make it one of the major health problems facing rabbit breeding today.

The only prophylactic measures currently effective in controlling this disease are realizable in intensive farms, or on wild rabbits bred in captivity.

However, direct prophylaxis is difficult to achieve due to the high virulence and spread of the virus.

In intensive farms, disease control is carried out by means of vaccination.

Such type of indirect prophylaxis involves the use of inactivated and adjuvanted virus vaccines, the preparation of which remains rather complex and closely linked to the availability of organs from animals which have already died of RHDV.

To address these issues at least in part, procedures have been developed to express the recombinant protein VP60in heterologous systems. More specifically, this recombinant protein is capable of self-assembly to form Virus Like Particles (VLPs) having an immunogenic power similar to that of the virus.

In particular, the VPLs produced to date in these systems have rather long and laborious purification procedures.

The purification procedures have extremely long operating times; the latter in fact provide for a plurality of methods of purification and/or concentration of the VLPs performed in series.

It is easy to appreciate how the need to carry out a plurality of purification methods in series can lead to a significant decrease in the yield of the purified product, i.e., of the recombinant protein.

To this must be added a further problem linked to the impossibility of carrying out monitoring on wild rabbits, which are a continuous source of virus.

### Description of the Invention

The main aim of the present invention is to provide a procedure for the production and purification of VP60 of RHDV that greatly facilitates the production process of same, simplifying its purification.

Within this aim, one object of the present invention is to provide a procedure for the production and purification which ensure adequate self-assembly to give stable VLPs.

This allows overcoming the aforementioned drawbacks of the prior art within the scope of a simple, rational, easy, efficient to use and cost-effective solution.

The aforementioned objects are achieved by the present procedure for the production and purification having the characteristics of claim 1.

### Brief Description of the Drawings

Other characteristics and advantages of the present invention will become more evident from the description of a preferred, but not claimed recombinant protein VP60 and related procedure for the production and purification, illustrated by way of an indicative, but non-limiting example, in the attached drawings in which:

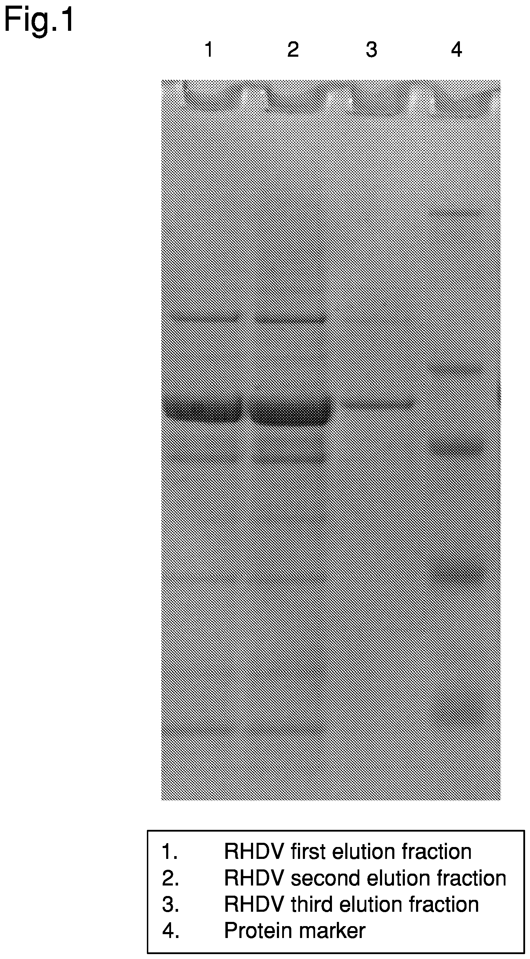

Figure 1 is a representation of the SDS-PAGE analysis performed on the R-VP60-His₇ fractions purified by immobilized metal affinity chromatography or IMAC.

### Embodiments of the Invention

Herein described but not claimed is a recombinant protein VP60 comprising an amino acid sequence presenting a polyhistidine tail at the carboxy-terminal end. In detail, it is an anti-RHDV recombinant protein VP60.

Preferably, the polyhistidine tail comprises seven histidine residues.

It cannot however be ruled out that the polyhistidine tail consists of eight, nine, etc. histidine residues.

Preferably, the recombinant protein VP60 has the following amino acid sequence (SEQ ID NO: 1).

This means that the anti-RHDV recombinant protein VP-60 according to the present invention has the following amino acid sequence (SEQ. ID NO:1)

MEGKARTAPQSDAAGTATTASYPGTTTDGMDPGVVATTSVVTTENSS

ASIATAGIGGPPQQVDQQETWRTNFYYNDVFTWSVADAPGSILYTVQH
SPQNNPFTAVLSQMYAGWAGGMQFRFIVAGSGVFGGRLVAAVIPPGIE
IGPGLEVRQFP
HVVIDARSLEPVTITMPDLRPNMYHPTGDPGLVPTLVLSVYNNLINPFG
GSTSAIQVTVETRPSEDFEFVMIRAPSSKTVDSISPAGLLTTPVLTGVGN
DNRWNGQIVGLQPVPGGFSTCNRHWNLNGSTYGWSSPRFADIDHRSG
SASYPGNNATNV
LQFWYANAGSAIDNPISQVAPDGFPDMSFVPFNGPGIPAAGWVGFGAI
WNSNSGAPNVTTVQAYELGFATGAPGNLQPTTNTSGAQTVAKSIYAV
VTGTGQSPAGLFVMASGIISTPNANAITYTPQPDRIVTTPGTPAAAPVG
KNTPIMFASVVRRTGDVNATAGSTNGTQYGTGSQPLPVTIGLSLNNYS
SALMPGQFFVWQLTFASGFMEIGLSVDGYFYAGTGASTTLIDLTELIDV
RPVGPRPSKSTLVFNLGGAANGFSYVGGHHHHHH
SEQ ID NO:1

The present invention relates to a procedure for the production and purification of the recombinant protein.

Such procedure comprises an isolation phase of a nucleotide sequence encoding for the protein VP60 from a viral strain of RHDV.

The viral strain is isolated from an outbreak of infection that occurred in a limited environment, in this case the Umbria-Marche territory.

However, alternative embodiments cannot be ruled out wherein the procedure according to the invention is applied to viral strains belonging to groups of subtypes such as RHDVa, RHDVb, RHDV2 and the so-called French variant. The procedure then continues with an extraction phase of viral RNA and reverse transcription of the viral RNA itself to obtain a sequence of cDNA.

The RNA extraction phase is performed starting from a sample according to what is known from the QIAamp^{®} Viral RNA Mini Handbook (QIAGEN) kit execution protocol.

According to a preferred embodiment of the procedure, the sample from which to extract the RNA is equal to about 5-10 mg of tissue, in this case 200 µL of liver, lung and spleen homogenate.

Once an RNA sample has been obtained, the extraction phase comprises an elution phase of the extracted RNA sample.

According to a preferred embodiment, the obtained RNA sample is eluted with 17 µL of H₂O RNAasi free.

From the 17 µL, 5 µL are taken and then denatured at a working temperature of substantially 65°C for an operating time of substantially 5 minutes.

This is followed by the retro-transcription phase which involves the use of start sequences (primers) chosen by the group comprising:
5'-TGTGAATTCTATAAATAATGGAGGGCAAAGCCCGC-3' (SEQ ID NO: 3),
5'-CAAAAGCTTCAGACATAAGAAAAGCC-3' (SEQ ID NO: 4).

Preferably, according to a preferred embodiment of the invention, the start nucleotide sequences comprise the above specific nucleotide sequences respectively for the restriction sites of EcoRI and HindIII: GAATTC and AAGCTT respectively.

More specifically, the complete start sequences for the respective EcoRI and HindIII restriction sites are shown below.

**Table 1**

| Primers | Sequence 5'→ 3' |
|---|---|
| RHD V/Ec oRI-F | TGTGAATTCTATAAATAATGGAGGGCAAAGC CCGC (SED ID NO:3) |
| RHD V/HindIII-R | CAAAAGCTTCAGACATAAGAAAAGCC (SEQ ID NO:4) |

Preferably, the retro-transcription phase takes place by means of a reverse transcriptase enzyme of the SuperScript ^{™} II Reverse Transcriptase of Invitrogen (Life technologies) type.

Afterwards, once a single-stranded cDNA sequence has been obtained, the procedure comprises a first amplification phase of the cDNA sequence itself. The first amplification phase is performed by setting up a first polymerase chain reaction, the so-called PCR, which, as the expert in the sector knows, involves the use of a DNA polymerase.

With reference to a preferred embodiment, the DNA polymerase used is of the Phusion High-Fidelity DNA Polymerase (Thermo Fisher) type.

In detail, the first amplification phase comprises a first plurality of operating phases which, in turn, comprise a first denaturation phase at a working temperature of substantially 95°C and for an operating time of substantially 30 seconds.

This is followed by the first amplification phase which comprises a first denaturation phase at a working temperature of substantially 95°C and for an operating time of substantially 5 seconds.

At this point, the first amplification phase comprises a first phase of adhesion of the start sequences to the cDNA filament at a working temperature of substantially 55°C for an operating time of substantially 15 seconds.

The start sequences (primers) used in the first adhesion phase are chosen from the group shown in Table 1.

Preferably, the start sequences (primers) used are RHDV/EcoRI-F in combination with RHDV/HindIII-R.

Furthermore, the first amplification phase comprises a first extension phase at a working temperature of substantially 72°C for an operating time of substantially 1 minute and 10 seconds.

More specifically, the first denaturation phase, the first adhesion phase and the first extension phase are repeated 30 times in sequence.

Finally, the procedure comprises a first phase of final extension at a working temperature of substantially 72°C and for an operating time of substantially 10 minutes to obtain the amplified nucleotide sequence.

At this point, the procedure comprises a first phase ligation of the amplified nucleotide sequences in a vector for transfer in baculovirus pOET2C-6xHis for the subsequent transformation of the competent cells of *E. Coli.*

The construct obtained and containing the gene encoding for the protein VP60 of RHDV was called RHDV-VP60/pOET2C.

It is pointed out that within the scope of the present treatise the term "gene" relates to a nucleotide sequence encoding for a protein.

To insert the polyhistidine tail at the C-terminal end of the protein VP60, a second amplification phase was performed of the 3' region encoding for the VP60 in frame with the nucleotide sequence encoding for the polyhistidine tail.

Similarly to the first amplification phase, the second amplification phase was performed by setting up a polymerase chain reaction, the so-called PCR, which, as the expert in the sector knows, provides for the use of a DNA polymerase.

Preferably, the DNA polymerase used is of the AccuPrime ^{™} *Pfx* DNA Polymerase (Life technologies) type.

In detail, the second amplification phase comprises a second plurality of operating phases which, in turn, comprise a second denaturation phase at a working temperature of substantially 95°C and for an operating time of substantially 2 minutes.

This is followed by the second amplification phase which comprises a second denaturation phase at a working temperature of substantially 95°C and for an operating time of substantially 15 seconds.

At this point, the second amplification phase comprises a second phase of adhesion of the cDNA filament start sequences at a working temperature of substantially 58°C for an operating time of substantially 30 seconds.

The start sequences (primers) used in the second adhesion phase are selected from the group shown in Table 2.

Preferably, the start sequences (primers) used are RHDV/Pst-F in combination with RHDV/His-R.

Preferably, according to a preferred embodiment of the invention, the start nucleotide sequences comprise the above specific nucleotide sequences respectively for the restriction sites of PstF and HindIII respectively: 5'-GTTGACTCAATTTCACCTGCAG -3', e 5'-GCATTTCGAATCAATGGTGGTGATGATGGTGACCTCCGACATAAGAA AAGCCATTAG-3' .

More specifically, the following table (Table 2) shows the complete nucleotide sequences for the respective restriction sites.

**Table 2**

| Primer | Sequence 5'→ 3' |
|---|---|
| RHDV/Pst-F | GTTGACTCAATTTCACCTGCAG (SEQ ID NO:5) |
| RHDV/His-R | GCATTTCGAATCAATGGTGGTGATGATGGTG ACCTCCGACATAAGAAAAGCCATTAG (SEQ ID NO:6) |

Furthermore, the second amplification phase comprises a second extension phase at a working temperature of substantially 68°C for an operating time of substantially 1 minute.

More specifically, the second denaturation phase, the second adhesion phase and the second extension phase are repeated 25 times in sequence.

Finally, the procedure comprises a third final extension phase at a working temperature of substantially 68°C and for an operating time of substantially 5 minutes to obtain the amplified nucleotide sequence.

At this point, the procedure comprises a phase of digestion of the amplified nucleotide sequence with the restriction enzymes PstI and HindIII, and related purification and recovery of same.

In other words, the digestion phase is aimed at the recovery of the 3' region of the VP60 gene in frame with the nucleotide sequence encoding for the polyhistidine tail.

At the same time, the RHDV-VP60/pOET2C construct was digested with the enzymes PstI HindIII and the linearized plasmid containing the 5' region of the VP60 gene was recovered.

The next phase involved a second ligation phase between the two products described above (region 3' and region 5'), and the subsequent transformation of the competent *E. Coli* cells.

The obtained construct, containing the gene encoding for the VP60 with the polyhistidine tail at the C-terminal end, has been called RHDV-VP60-His₇/pOET2C.

It is pointed out that in the present treatise the term "gene" relates to a nucleotide sequence encoding for a protein.

It may, but does not necessarily, comprise elements found in and/or associated with a gene encoding for that protein in nature.

Below is the sequence analysis carried out on RHDV-VP60-His₇.

ATGGAGGGCAAAGCCCGCACAGCGCCGCAAAGCGATGCAGCGGGC

ACTGCTACCACAGCATCAGTCCCTGGAACCACAACCGACGGTATGG
ATCCTGGCGTCGTGGCCACAACCAGCGTGGTCACCACCGAGAATTC
ATCCGCGTCGATTGCAACGGCAGGGATTGGCGGACCACCCCAACAG
GTGGACCAACAAGAGACATGGAGAACAAATTTTTATTACAATGATG
TTTTCACTTGGTCAGTCGCAGACGCCCCCGGCAGTATACTTTATACC
GTCCAGCATTCTCCACAAAACAACCCATTTACAGCCGTACTGAGCC
AGATGTACGCTGGCTGGGCTGGTGGCATGCAGTTTCGCTTCATAGTT
GCTGGTTCAGGTGTGTTCGGTGGGCGACTGGTTGCAGCTGTGATAC
CACCAGGCATTGAGATTGGACCAGGGTTGGAGGTCAGGCAATTTCC
TCATGTTGTCATCGACGCCCGTTCACTTGAACCTGTTACCATCACCA
TGCCAGACTTGCGTCCCAACATGTACCATCCAACTGGTGACCCTGGT
CTTGTCCCCACACTGGTCCTTAGTGTTTACAACAATCTCATCAACCC
GTTTGGAGGGTCCACCAGCGCAATCCAGGTGACGGTGGAAACAAG
GCCGAGTGAAGACTTTGAGTTCGTGATGATTCGAGCCCCTTCTAGC
AAGACTGTTGACTCAATTTCACCTGCAGGCCTCCTCACAACCCCAGT
TCTCACCGGGGTCGGTAATGACAACAGGTGGAACGGCCAAATAGTG
GGACTGCAACCGGTGCCCGGAGGGTTTTCTACATGCAACAGGCATT
GGAACTTGAATGGCAGCACGTATGGCTGGTCAAGCCCTCGGTTTGC
TGACATTGACCATCGAAGTGGCAGTGCAAGTTACCCTGGGAACAAC
GCAACCAACGTGCTTCAGTTTTGGTATGCCAACGCTGGATCTGCAAT
TGACAACCCCATCTCCCAGGTTGCACCGGACGGCTTTCCTGACATGT
CGTTCGTGCCCTTTAATGGTCCTGGCATCCCAGCCGCGGGGTGGGTC
GGATTTGGTGCAATCTGGAACAGCAACAGCGGTGCTCCCAACGTTA
CGACTGTGCAGGCCTATGAGTTAGGTTTTGCCACTGGGGCACCAGG
CAACCTCCAGCCCACTACCAACACTTCAGGTGCGCAAACTGTCGCC
AAATCCATTTACGCCGTGGTAACCGGTACAGGCCAAAGCCCAGCCG
GGTTGTTTGTGATGGCCTCGGGCATCATCTCTACCCCAAATGCCAAT
GCCATCACATACACACCTCAACCAGACAGAATTGTAACCACACCCG
GCACACCAGCCGCTGCACCTGTGGGTAAAAACACACCCATCATGTT
TGCGTCTGTCGTTAGGCGCACCGGTGACGTTAACGCCACAGCTGGA
TCAACTAACGGCACTCAGTATGGCACAGGCTCCCAACCACTGCCAG
TGACAATTGGGCTTTCGCTCAACAACTACTCGTCAGCACTCATGCCC
GGACAGTTCTTCGTGTGGCAGTTAACCTTTGCATCTGGTTTCATGGA
GATTGGTTTAAGTGTGGACGGGTATTTTTATGCAGGAACAGGGGCC
TCAACCACACTCATTGACTTGACTGAACTCATTGATGTTCGCCCTGT
GGGACCCAGGCCATCCAAAAGCACACTCGTGTTCAACCTGGGGGGC
GCAGCTAATGGCTTTTCTTATGTCGGAGGTCACCATCATCACCACCA
TCACTAG

SEQ ID NO:2

Also described but not claimed is a transfer vector encoding for the protein VP60.

Advantageously, the nucleotide sequence consists in SEQ ID NO:2.

Preferably, the nucleotide sequence is cloned in a transfer vector for baculovirus of the type of baculovirus transfer vector pOET2C-6xHis.

More specifically, the above transfer vector comprises a nucleotide sequence containing specific sequences of Baculovirus and is optimized for transformation into E. Coli.

Furthermore, the procedure comprises an expression and synthesis phase of the protein R-VP60-His₇.

In detail, the plasmid RHDV-VP60-His/Poet2C was used for the generation of the recombinant baculoviruses expressing the recombinant protein R-VP60-His₇.

The expression and synthesis phase comprises a transfection phase of Sf21 insect cells grown in monolayer on a HYQ SFX-Insect serum-free medium (Hyclone).

The Sf21 insect cells were transfected with linearized flashBAC^{™} DNA and plasmid DNA RHDV-VP60-His/Poet2C according to what is known from the Oxford Expression Technologies' execution protocol.

In the case in question, the Sf21 insect cells were transfected with 100 ng of linearized flashBAC^{™} DNA and 500 ng of RHDV-VP60-His/Poet2C DNA.

After a 5-day incubation period at 27°C, the cell supernatant containing the recombinant baculovirus (P1 viral stock) was collected and used for the production of a small-scale high titre viral stock (P2 viral stock).

P2 viral stock was subsequently used for the production of a large-scale high titre viral stock (P3 viral stock).

At this point, the expression and synthesis phase comprises a production phase of the recombinant protein R-VP60-His₇.

The production phase comprises an infection phase of Sf21 insect cells with the previously obtained recombinant baculovirus, i.e. obtained from the transfection phase.

The production phase takes place by means of the culture of Sf21 insect cells inside rotating bottles in serum-free ExCell medium (SIGMA) with the addition of 100 µg/ml of streptomycin, 0.25 µg/ml of amphotericin B and 10 µg/ml of gentamycin.

In detail, the Sf21 insect cells are sown inside a number of rotating bottles at a concentration of 1.1 × 10⁶ cells/ml (200 × 10⁶ cells in 180 ml of ExCell Medium per rotating bottle) and infected with 0.1-1 recombinant baculovirus plaque forming units (PFUs) per cell.

Subsequently, the production phase comprises an incubation phase for an operating time of substantially three days.

At the end of the three days, the infected cells contained in the medium and the cells grown in adhesion to the wall of the rotating bottles are collected.

For each rotating bottle used, the medium containing the cells in suspension is then divided into 4x50 ml falcon tubes, and centrifuged at about 300 × g at a working temperature of substantially +4°C, and for an operating time of substantially 10 minutes.

Instead, the cells attached to the wall of the rotating bottles are washed with 10 ml of PBS.

Afterwards, the supernatant is eliminated and the cell pellets are washed with the 10 ml of PBS recovered from the washing of the cells attached onto the rotating bottles.

At this point, the cells are centrifuged again at about 300 × g at a working temperature of substantially 4°C for an operating time of substantially 7 minutes. After which the PBS is removed and the cells undergo lysis.

In this regard, it is specified that the present invention also relates to a heterologous system of expression of the prokaryotic or eukaryotic type.

Preferably, the heterologous expression system of the prokaryotic type comprises vectors selected from the group including *E. Coli and Bacillus subtilitis.*

Alternatively, advantageously, the heterologous expression system of the eukaryotic type comprises vectors selected from the group including: yeasts such as e.g. *S. cerevisiae and Pichia pastoris,* or culture cells such as e.g. insect and mammal cells, or else plant models.

Preferably, the expression vector is of the recombinant Baculovirus type.

Finally, once the expression of the recombinant protein has been obtained, the procedure comprises a phase of extraction and purification of same.

The extraction and purification phase of the protein R-VP60-His₇ comprises a lysis phase wherein the infected Sf21 cells attached onto the walls of the rotating bottles are suspended in a specific 10 ml lysis buffer.

In detail, the lysis buffer comprises 50 mM NaH₂PO₄/Na₂HPO₄ at pH8, 300 mM NaCl, 1% Triton X-100.

Afterwards, to the cell pellets contained in the 4 falcon tubes, a further lysis buffer is added for a total of another 10 ml.

At this point, the two cell lysate fractions are combined in a single tube for a total of 20 ml of lysate.

The fact is underlined that the cell lysates obtained by the union of the two fractions can be stored at a temperature of substantially -20°C until the next phase of the procedure.

In this respect, after the lysis phase, the procedure comprises a phase of extraction of the proteins R-VP60-His₇.

The cell lysate undergoes centrifugation at 16000 × g at a working temperature of substantially 4°C and for an operating time of substantially 30 minutes.

The next step is the recovery of the supernatant and the purification phase.

In detail, the purification phase is carried out by means of an affinity chromatography.

Preferably, the lysate is purified by means of only one affinity chromatography. Advantageously, the affinity chromatography is of the IMAC type.

In other words, the recombinant protein R-VP60-His₇ is purified by a single passage through the chromatographic column described below.

In fact, the preparation of the above chromatography column provides for the use of an agarose granule matrix (6%) charged with Nickel ions specifically binding the histidines of the proteins (HIS-Select^{®} HF Nickel Affinity Gel,

SIGMA ALDRICH).

Purification is performed according to what is known from the HIS-Select^{®} HF Nickel Affinity Gel (SIGMA ALDRICH) execution protocol under native conditions.

Such execution protocol is, furthermore, implemented by means of the following solutions:
- the final bed of the column of 0.25 ml (per number of cells deriving from a rotating bottle), this indicates the amount of resin used for each rotating bottle; and
- addition of imidazole to the cell lysate in a final concentration of 10 mM. Figure 1 shows the unexpected results relating to the qualitative and quantitative aspects of the recombinant protein R-VP60-His₇ purified by means of the above affinity chromatography.

Figure 1 shows all the purified protein fractions. In particular, the obtained protein fractions underwent electrophoresis on acrylamide gels under denaturing conditions (SDS-PAGE), and were subsequently dyed with Coomassie-Blu.

More specifically: lines 1, 2 and 3 represent the first, second and third elution fractions of the purified recombinant protein R-VP60-His₇, respectively; finally, line 4 indicates the protein marker for determining the molecular weight of the purified molecule.

The speed and ease of application of the procedure to which the present invention relates, ensures the production and purification of the R-VP60-His₇ also in a scale-up plant aimed at the large-scale production of the recombinant protein R-VP60-His₇ itself.

To this must be added that, besides the quantitative aspect inherent in the high yield of the product obtained starting from the cell lysate, the high degree of purity of the R-VP60-His₇ is evident.

At this point, the extraction and purification phase comprises a dialysis phase at a pH value between 4.5 and 5.5 with a solution comprising 140 mM NaCl, 2.68 mM KCl, 1.7 mM KH₂PO₄, 1.8 mM Na₂HPO₄, 9 mM NaH₂PO₄ (H₂0).

In particular, the fact is underlined that the use of solutions having a pH higher than 6.0 hinders the formation of VLP.

By contrast, pH values between 4.5 and 5.5 promote the formation of stable VLPs.

Furthermore, it is noticed that the fact that R-VP60-His₇ self-assembles to form VLPs makes possible its use in pharmaceutical compositions for vaccine purposes.

Also described but not claimed is a pharmaceutical composition comprising Virus Like Particles (VLPs) obtained from the nucleic acid molecule described above, and more specifically from the self-assembly of the recombinant proteins R-VP60-His₇.

It has in practice been ascertained how the described invention achieves the intended objects.

The fact is underlined that the special solution of providing a polyhistidine tail at the carboxy-terminal end of the recombinant protein R-VP60-His₇ makes it possible to purify the latter by means of only one affinity chromatography, thus considerably increasing the final purification yield.

## Claims

1. Procedure for the production and purification of a recombinant protein VP60 from a Rabbit Hemorrhagic Disease Virus (RHDV) comprising an amino acid sequence presenting a polyhistidine tail at the carboxy-terminal end, the procedure comprises at least the following phases:
- isolation of a nucleotide sequence coding for VP60 from a viral strain of RHDV, in turn, comprising the steps of:
- extraction of viral RNA and of reverse transcription of said viral RNA to obtain a sequence of cDNA;
- at least an amplification of said sequence of cDNA;
- cloning of said amplified sequence of cDNA in a transfer vector encoding said polyhistidine tail;
- expression and synthesis of said recombinant protein; and
- extraction and purification of said recombinant protein;
said extraction and purification phase comprises an affinity chromatography wherein said extraction and purification phase comprises at least one dialysis phase at a pH value substantially equal to 5.5 with a solution comprising NaCl, KCl, KH2PO4, Na2HPO4, NaH2PO4 and HCl.

2. Procedure according to claim 1, **characterized by** the fact that said polyhistidine tail comprises seven histidine residues.

3. Procedure according to any of the preceding claims, **characterized by** the fact that said recombinant protein presents the amino acid sequence SEQ ID NO:1.

4. Procedure according to any of the preceding claims, **characterized by** the fact that said recombinant protein is encoded from a transfer vector.

5. Procedure according to any of the preceding claims, **characterized by** the fact said transfer vector comprises a nucleotide sequence consisting in SEQ ID NO:2.

6. Procedure according to any of the preceding claim, **characterized by** the fact that said nucleotide sequence is cloned in a baculovirus transfer vector pOET2C-7xHis.

7. Procedure according to claim 1, **characterised by** the fact that said affinity chromatography is of the IMAC type.

8. Procedure according to one or more of the preceding claims, **characterised by** the fact that said first amplification phase is mediated by at least one start sequence selected from the list comprising: 5'-TGTGAATTCTATAAATAATGGAGGGCAAAGCCCGC-3', 5 ' -C AAAAGCTTC AG AC ATAAGAAAAGCC-3 ', 5'-GTTGACTCAATTTCACCTGCAG-3', 5'-GCATTTCGAATCAATGGTGGTGATGATGGTGACCTCCGACATAAGAA AAGCCATTAG-3'.

## Patentansprüche

1. Verfahren zur Herstellung und Reinigung eines rekombinanten Proteins VP60 aus einem Virus der hämorrhagischen Kaninchenkrankheit (RHDV), das eine Aminosäuresequenz mit einem Polyhistidinschwanz am carboxyterminalen Ende aufweist, wobei das Verfahren mindestens die folgenden Phasen umfasst:
- Isolierung einer Nukleotidsequenz, die für VP60 kodiert, aus einem Virusstamm von RHDV, die wiederum die folgenden Schritte umfasst:
o Extraktion der viralen RNA und reverse Transkription der viralen RNA, um eine cDNA-Sequenz zu erhalten;
o zumindest eine Amplifikation der cDNA-Sequenz;
- Klonierung der amplifizierten cDNA-Sequenz in einem Transfervektor, der für den Polyhistidinschwanz kodiert;
- Expression und Synthese des rekombinanten Proteins; und
- Extraktion und Reinigung des rekombinanten Proteins;
die Extraktions- und Reinigungsphase umfasst eine Affinitätschromatographie, wobei die Extraktions- und Reinigungsphase mindestens eine Dialysephase bei einem pH-Wert von im Wesentlichen gleich 5,5 mit einer Lösung umfasst, die NaCl, KCl, KH2P04, Na2HPO4, NaH2P04 und HCl umfasst.

2. Verfahren nach Anspruch 1, **dadurch gekennzeichnet, dass** der Polyhistidinschwanz sieben Histidinreste umfasst.

3. Verfahren nach einem der vorhergehenden Ansprüche, **dadurch gekennzeichnet, dass** das rekombinante Protein die Aminosäuresequenz SEQ ID NO:1 aufweist.

4. Verfahren nach einem der vorangehenden Ansprüche, **dadurch gekennzeichnet, dass** das rekombinante Protein von einem Transfervektor kodiert wird.

5. Verfahren nach einem der vorangehenden Ansprüche, **dadurch gekennzeichnet, dass** der Transfervektor eine Nukleotidsequenz, bestehend aus SEQ ID NO:2, umfasst.

6. Verfahren nach einem der vorangehenden Ansprüche, **dadurch gekennzeichnet, dass** die Nukleotidsequenz in einem Baculovirus-Transfervektor pOET2C-7xHis kloniert wird.

7. Verfahren nach Anspruch 1, **gekennzeichnet durch** die Tatsache, dass die Affinitätschromatographie vom IMAC-Typ ist.

8. Verfahren nach einem oder mehreren der vorhergehenden Ansprüche, **dadurch gekennzeichnet, dass** die erste Amplifikationsphase durch mindestens eine Startsequenz vermittelt wird, die aus der Liste ausgewählt ist, die umfasst:
5'-TGTGAATTCTATAAATAATGGAGGGCAAAGCCCGC-3',
5'-CAAAAGCTTCAGACATAAGAAAAGCC-3',
5'-GTTGACTCAATTTCACCTGCAG-3',

5'-GCATTTCGAATCAATGGTGGTGATGATGGTGACCTCCGACATAAGAA
AAGCCATTAG-3'.

## Revendications

1. - Procédé de production et de purification d'une protéine recombinante VP60 provenant d'un virus de la maladie hémorragique du lapin (RHDV) comprenant une séquence d'acides aminés présentant une queue polyhistidine au niveau de l'extrémité carboxy-terminale, le procédé comprenant au moins les phases suivantes :
- isolement d'une séquence nucléotidique codant pour VP60 à partir d'une souche virale de RHDV, à son tour, comprenant les étapes de :
- extraction d'ARN viral et transcription inverse dudit ARN viral pour obtenir une séquence d'ADNc ;
- au moins une amplification de ladite séquence d'ADNc ;
- clonage de ladite séquence amplifiée d'ADNc dans un vecteur de transfert codant pour ladite queue polyhistidine ;
- expression et synthèse de ladite protéine recombinante ; et
- extraction et purification de ladite protéine recombinante ;
ladite phase d'extraction et de purification comprend une chromatographie d'affinité,
dans lequel ladite phase d'extraction et de purification comprend au moins une phase de dialyse à une valeur de pH sensiblement égale à 5,5 avec une solution comprenant NaCl, KCl, KH2PO4, Na2HPO4, NaH2PO4 et HCl.

2. - Procédé selon la revendication 1, **caractérisé par le fait que** ladite queue polyhistidine comprend sept résidus histidine.

3. - Procédé selon l'une quelconque des revendications précédentes, **caractérisé par le fait que** ladite protéine recombinante présente la séquence d'acides aminés SEQ ID NO : 1.

4. - Procédé selon l'une quelconque des revendications précédentes, **caractérisé par le fait que** ladite protéine recombinante est codée à partir d'un vecteur de transfert.

5. - Procédé selon l'une quelconque des revendications précédentes, **caractérisé par le fait que** ledit vecteur de transfert comprend une séquence nucléotidique consistant en SEQ ID NO : 2.

6. - Procédé selon l'une quelconque des revendications précédentes, **caractérisé par le fait que** ladite séquence nucléotidique est clonée dans un vecteur de transfert de baculovirus pOET2C-7xHis.

7. - Procédé selon la revendication 1, **caractérisé par le fait que** ladite chromatographie d'affinité est de type IMAC.

8. - Procédé selon une ou plusieurs des revendications précédentes, **caractérisé par le fait que** ladite première phase d'amplification est médiée par au moins une séquence de départ choisie dans la liste comprenant : 5'-TGTGAATTCTATAAATAATGGAGGGCAAAGCCCGC-3', 5'-CAAAAGCTTCAGACATAAGAAAAGCC-3', 5'-GTTGACTCAATTTCACCTGCAG-3',
